(19) Europäisches Patentamt
European Patent Office
Office européen des brevets

(11) **EP 4 744 702 A1**

(12) **EUROPEAN PATENT APPLICATION**

(43) Date of publication:
**20.05.2026 Bulletin 2026/21**

(21) Application number: **25214778.0**

(22) Date of filing: **11.11.2025**

(51) International Patent Classification (IPC):
**A61M 16/00** (2006.01)   **G16H 20/00** (2018.01)
**A61B 5/08** (2006.01)    **A61M 16/06** (2006.01)

(52) Cooperative Patent Classification (CPC):
**A61M 16/024; A61B 5/085; A61B 5/091;**
**A61B 5/113; A61B 5/4836; G16H 10/60;**
**G16H 20/40; G16H 30/20; G16H 30/40;**
**G16H 40/63; G16H 40/67; G16H 50/50;**
**G16H 50/70;** A61B 5/1114; A61B 5/6803;    (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH LA MA MD TN**

(30) Priority: **14.11.2024 US 202463720357 P**

(71) Applicant: **Koninklijke Philips N.V.**
**5656 AE Eindhoven (NL)**

(72) Inventors:
• **HENDRIKS, Cornelis Petrus**
**5656 AG Eindhoven (NL)**

• **BUIZZA, Roberto**
**5656 AG Eindhoven (NL)**
• **VAN ZANTEN, Joyce**
**5656 AG Eindhoven (NL)**
• **DE VRIES, Jan Johannes Gerardus**
**5656 AG Eindhoven (NL)**
• **SCHIPPER, Alphonsus Tarcisius Jozef Maria**
**5656 AG Eindhoven (NL)**

(74) Representative: **Philips Intellectual Property &**
**Standards**
**High Tech Campus 52**
**5656 AG Eindhoven (NL)**

(54) **SYSTEMS AND METHODS FOR DETERMINING CHEEK IMPEDANCE DURING MECHANICAL VENTILATION**

(57)    Systems (10) and methods (100) for determining airway impedance of a patient undergoing mechanical ventilation are disclosed. A controller (22) including one or more electronic processors (30) is configured to: receive (104) an apparent airway impedance of a patient measured during an impedance estimation procedure by a mechanical ventilator (14); determine (106) at least one cheek metric based on data received from at least one cheek sensor (24a, 24b, 24c) associated with the patient; determine (108) a cheek impedance based on the at least one cheek metric; and determine (110) a true airway impedance based on the apparent airway impedance and the cheek impedance.

FIG. 3

(Cont. next page)

(52) Cooperative Patent Classification (CPC): (Cont.)
A61B 5/7264; A61M 16/0006; A61M 16/06;
A61M 2016/0027; A61M 2205/3306;
A61M 2205/332; A61M 2205/3375;
A61M 2205/3592; A61M 2230/46

**Description**

FIELD OF THE INVENTION

The invention relates to systems and methods for determining the effect of cheek inflation during non-invasive mechanical ventilation, and more particularly for determining cheek impedance when diagnosing and treating expiratory flow limitation (EFL) in chronic obstructive pulmonary disease (COPD) patients.

BACKGROUND OF THE INVENTION

In patients with COPD, airflow obstruction requires developing greater respiratory pressures to produce pulmonary ventilation. These patients experience respiratory muscle fatigue and, in severe conditions, respiratory failure. By increasing lung volumes, a patient can reduce airway resistance and increase respiratory flows. Breathing at this abnormal operating point (dynamic hyperinflation and intrinsic positive end expiratory pressure, or "iPEEP") requires extra work, especially if the situation is worsened by EFL. It has been shown, both in physiologic and clinical studies, that the application of an external PEEP by means of mechanical ventilators or CPAP devices reduces the work of breathing, normalizes the breathing pattern, improves blood gases, and reduces patient-ventilator asynchrony.

The Philips BiPAP A40 EFL ventilator, for example, uses a forced oscillation technique (FOT) to detect EFL. Other methods for EFL detection, specifically for intubated patients in the ICU, rely on: (a) the measurements of expiratory flow during a specific maneuver, i.e., during an intentional change of expiratory driving pressure, system resistance, or interruption of the flow; or (b) on the analysis of the expiratory resistance in the absence of a spontaneous effort - obtained via mathematical analysis of the flow waveforms. *See, e.g.,* Junhasavasdikul, D. et al., 2018, Expiratory Flow Limitation During Mechanical Ventilation, CHEST 2018; 154(4):948-962; Junhasavasdikul, D., et al., 2024, Expiratory flow limitation during mechanical ventilation: real-time detection and physiological subtypes, Crit Care 28, 171 (2024).

A specific challenge in measuring respiratory parameters, such as EFL, is the influence of cheek inflation. In the detection of EFL via FOT, the inflation of the cheeks significantly influences the measurement of airway impedance. Prior art has described how this influence can be reduced by manually supporting the cheeks of the patient during the FOT measurement-i.e., keeping the cheeks firmly pressed against the patient's teeth to minimize cheek inflation. *See, e.g.,* Dellaca R.L., et al., 2004, Detection of expiratory flow limitation in COPD using the forced oscillation technique, Eur. Respir J 2004; 23: 232-240; Michaelson, E.D. et al., 1975 "Pulmonary Mechanics by Spectral Analysis of Forced Random Noise", The Journal of Clinical Investigation Volume 56 November 1975.1210-1230. Alternative approaches have sought to measure cheek impedance by using an actuation with a second device-e.g., a syringe or loudspeaker-and factoring this measurement into overall airway impedance estimate. *See, e.g.,* Jaeger, M.J., 1982, Effect Of The Cheeks And The Compliance Of Alveolar Gas On The Measurement Of Respiratory Variables, Respiration Physiology (1982) 47,325 340; Clements, J.A. et al., 1959, Estimation Of Pulmonary Resistance By Repetitive Interruption Of Airflow, J Clin Invest. 1959 Jul; 38(7):1262-70. Such efforts have showed that respiratory resistance measured via FOT can be underestimated by 10-50% if the effect of cheek inflation is ignored.

Even if a patient's cheek impedance has been measured at a particular point in time, such as during a hospital stay or during an expert in-home visit, the cheek impedance may vary over time. Moreover, any single cheek impedance measurement is sensitive to the patient's condition (e.g., neck and oral muscle tension), and may fluctuate even over short time periods.

Particularly in the home setting, during non-invasive ventilation (NIV), continuous and consistent manual cheek support is infeasible, not to mention uncomfortable for the patient. It is also not possible to use a second actuating device which needs to be operated by an expert. But if the cheek inflation is ignored, an EFL detection algorithm would be inaccurate and hence the therapy device may initiate a sub-optimal intervention, such as a new PEEP setting that lessens the therapeutic benefit for the patient. Therefore, there is an unmet need to determine and compensate for cheek inflation in NIV-EFL home therapy.

SUMMARY OF THE INVENTION

It is an object of the invention to provide systems and methods for determining cheek impedance so that EFL diagnosis and treatment reflects proper compensation for cheek inflation.

Another object is to make the process of cheek impedance measurement automatic, requiring no support from a patient or other operator to manually restrict cheek deformation.

Another object is to make the process of cheek impedance measurement unobtrusive, requiring no cheek compression and no particular patient cooperation relative to cheek inflation.

Another object is to make the process of cheek impedance measurement easy to perform, making use of information available in the normal workflow of NIV therapy.

Another object is to make the process of cheek impedance measurement simple, involving no specialized cheek measuring device requiring operation or control by an expert.

Another object is to make the process of cheek impedance measurement accurate, involving continuous measurement to reduce variability over time and during changes in patient condition.

The invention is essentially defined by the independent claims. Advantageous embodiments are defined in the dependent claims.

In an aspect of the invention, a system is provided for determining airway impedance of a patient undergoing mechanical ventilation, where the system includes a controller having one or more electronic processors. The controller is configured to receive an apparent airway impedance of a patient measured during an impedance estimation procedure by a mechanical ventilator; determine at least one cheek metric based on data received from at least one cheek sensor associated with the patient; determine a cheek impedance based on the at least one cheek metric; and determine a true airway impedance based on the apparent airway impedance and the cheek impedance.

In certain embodiments, the at least one cheek sensor is a microphone and/or a PPG sensor. In certain embodiments, at least one cheek metric is a resonance frequency and/or a change in volume.

In certain embodiments, the at least one cheek sensor includes an imaging device and the at least one cheek metric includes at least one cheek geometry parameter. In certain embodiments, the controller is configured to determine cheek impedance based on a biomechanical model, where the biomechanical model is based on the at least one cheek geometry parameter.

In certain embodiments, the controller is configured to recommend a change to at least one ventilation parameter based on the true airway impedance. In certain embodiments, the change to at least one ventilation parameter is an increase or decrease in positive end expiratory pressure (PEEP), and/or an increase or decrease in respiratory rate.

According to another aspect of the invention, there is provided a method for determining airway impedance of a patient undergoing mechanical ventilation. The method is implemented by a controller including one or more electronic processors performing the steps of: receiving an apparent airway impedance of a patient measured while the patient is undergoing an impedance estimation procedure by a mechanical ventilator; receiving data from at least one cheek sensor associated with the patient; determining at least one cheek metric based on the data of at least one cheek sensor; determining a cheek impedance based on the at least one cheek metric; and determining a true airway impedance based on the apparent airway impedance and the cheek impedance.

In certain embodiments, the method also includes the step of determining an expiratory flow limitation (EFL) status based on the true airway impedance. In an embodiment, the method also includes the step of outputting a recommended change in positive end expiratory pressure (PEEP) level based on the EFL status.

In certain embodiments, the at least one cheek sensor includes a microphone and the cheek metric includes a resonance frequency.

In certain embodiments, the at least one cheek sensor includes a PPG sensor, and the cheek metric includes a change in volume.

In certain embodiments, the at least one cheek sensor includes an imaging device, the at least one cheek metric includes at least one cheek geometry parameter, and the cheek impedance is determined based on a biomechanical model, wherein the biomechanical model is based on the at least one cheek geometry parameter.

These and other aspects of the invention will be apparent from and elucidated with reference to the embodiments described hereinafter.

One advantage of the invention resides in being able to determine cheek impedance in non-clinical and non-hospital settings, such as in the home.

Another advantage resides in being able to determine cheek impedance without the use of a separate, dedicated device or maneuver requiring expert operation and interpretation.

Another advantage resides in being able to determine cheek impedance without active participation from the patient relative to cheek compression or inflation.

Another advantage resides in being able to continuously determine cheek impedance over time and during changes in patient condition.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The invention may take form in various components and arrangements of components, and in various steps and arrangements of steps. The drawings are only for purposes of illustrating exemplary embodiments and are not to be construed as limiting the invention.

FIGURE 1 is a diagram of a system according to embodiments of the invention.
FIGURE 2 is a conceptual schematic diagram of the system in Fig. 1.
FIGURE 3 is a flowchart of a method according to embodiments of the invention.
FIGURE 4 is a flowchart of a method according to embodiments of the invention.
FIGURE 5 is a flowchart detailing a method resulting in step 107 in Fig. 4, according to embodiments of the invention.
FIGURE 6 is a diagram of a computer system for use in embodiments of the invention.

DESCRIPTION OF EMBODIMENTS

The invention will be described herein with reference to the Figures. It should be understood that the description and specific examples provided, while indicating exemplary embodiments, are intended for purposes of illustration only, and not intended to limit the scope of the invention. It should also be understood that the figures are mere schematics and not drawn to scale. It should also be understood that the same reference numerals are used throughout the Figures to indicate the same or similar parts.

In the context of the invention, impedance is the relation between pressure and flow changes during oscillatory flow in and out of the lungs. Impedance depends on the frequency of oscillatory flow, as well as the compliance (or elastance), resistance, and inertance of the different parts of the respiratory system. More precisely, respiratory system impedance ($Z_{rs}$) has two basic components: resistance ($R_{rs}$) and reactance ($X_{rs}$)-where:

$$Z_{rs} = R_{rs} + iX_{rs} \; (i \text{ is } \sqrt{-1};$$

the imaginary number unit)
And the $X_{rs}$ component of $Z_{rs}$ can be further defined as:

$$Z_{rs} = R_{rs} + i(\omega I_{rs} - E_{rs}/\omega)$$

where $I_{rs}$ is respiratory system inertance, $E_{rs}$ is respiratory system elastance, and $\omega$ is $2\pi f$ oscillation frequency.
In EFL detection, the reactance during inhalation and exhalation is determined; ideally only of the respiratory system, so excluding the cheeks. Although deformation and movement of air contained in the mouth may also influence such reactance measurements, this effect seems negligible compared to the cheek impedance and so it is not separately accounted for herein.
The invention generally includes systems and methods for determining cheek impedance, where:
Compliance C (ml cm $H_2O$ $^{-1}$) is the resistance against deformation (inverse value of the cheek elasticity);

- Resistance R (cm $H_2O$ L $^{-1}$ sec) is the opposition against movement (viscous resistance of the cheek tissue); and
- Inertance I (cm $H_2O$ L $^{-1}$ sec$^2$) is the opposition against a change in movement (mass of the cheek tissue)

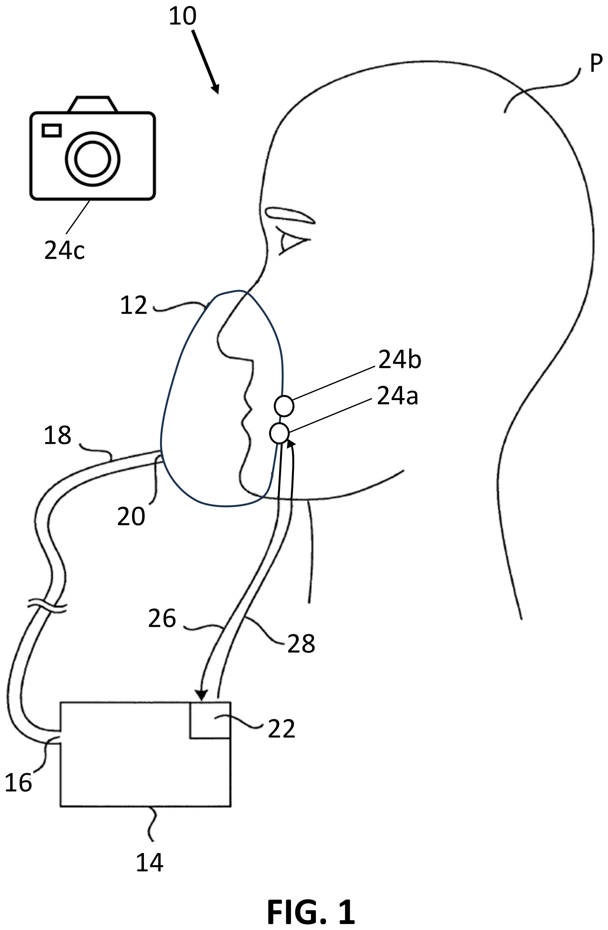

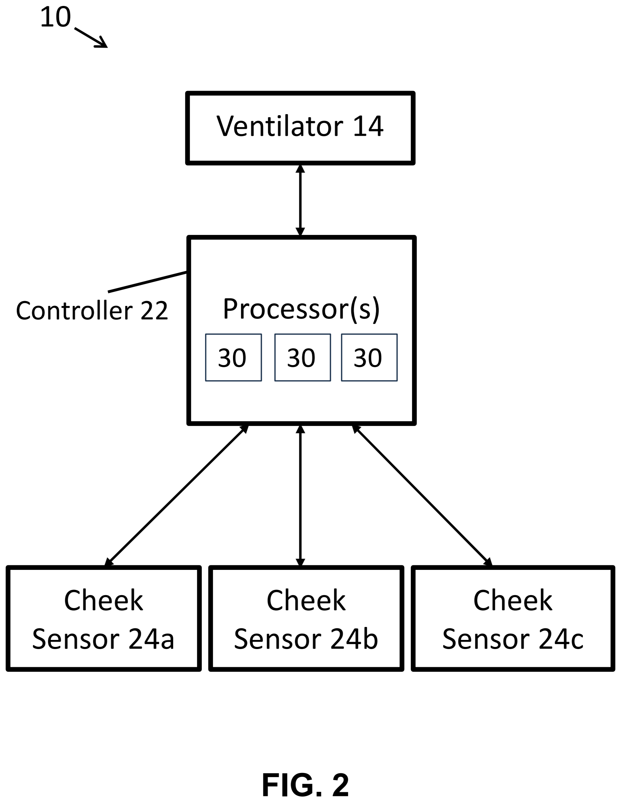

FIGURE 1 displays a schematic of a system 10 according to an embodiment of the present invention. A patient P is wearing a patient interface 12 that is connected to a mechanical ventilator 14, such as a continuous positive airway pressure (CPAP) device, Bi-level positive airway pressure (BiPAP) device, non-invasive ventilator, acute care ventilator, or any other suitable device for providing breathing or airway pressure support. The ventilator 14 provides a pressurized stream of breathable gas via outlet 16, through circuit conduit 18, and then into the patient interface 12 via opening 20. The patient interface 12 depicted is a full face mask type, covering the patient's entire nasal and oral cavities.
Operation of the ventilator 14 is managed by controller 22, which includes one or more processors 30 (not shown in Fig. 1). The controller is capable of receiving and transmitting data, following or providing machine-readable instructions, performing calculations, and the like.
Associated with the patient are cheek sensors 24a, 24b, and 24c. Sensors 24a and 24b are shown as embedded, attached to, or otherwise associated with the patient interface 12, essentially adjacent to the patient's cheek. Sensor 24a is an acoustic sensor such as a microphone. Sensor 24b is a photoplethysmography (PPG) sensor. Sensor 24c is shown as proximate to the patient but separated from the patient interface 12. Sensor 24c is an imaging device capable of imaging the patient's face, including at least one or both of the patient's cheeks and/or surrounding anatomy. Sensor 24c may be a dedicated medical imaging device such as an ultrasound probe, an x-ray detector, or an MRI or CT scanner, or sensor 24c may be a non-medical imaging device such as a digital camera, smartphone or tablet camera, 3D camera, or 3D scanner. In all cases, the sensors 24a-c are associated with the patient, and in particular the patient's cheek(s), because they are positioned and configured to detect features and attributes of the patient's cheek(s), as detailed further below.
As shown in Fig. 1, the controller 22 is electrically coupled to cheek sensor 24a via wires 26 and 28 so as to receive and send data, signals, instructions, etc. from and to the cheek sensor 24a. Cheek sensors 24b and 24c can likewise be physically wired to the controller (not shown). Alternatively, any of cheek sensors 24a, 24b, and 24c may be in wireless communication with the controller 22 via WiFi, Bluetooth, near-field communication, RFID, or other suitable wireless communication methodologies.
While only one of each cheek sensor 24a-c is shown in Fig. 1, multiple of each type may be utilized within the scope of the invention. For example, any cheek sensor 24a, 24b, or 24c may have a counterpart placed on the other side of the patient P and/or patient interface 12 so as to both sense information (e.g., simultaneously) relating to both cheeks. Similarly, each of the types of cheek sensors 24a, 24b, and 24c may be combined with any other, whether on the same side of the patient P or patient interface 12 or otherwise.
Although the controller 22 is depicted as part of the ventilator 14 in Fig. 1, the controller 22 may be located on (or have its processors distributed among) one or more other devices, such as one of the cheek sensors 24a-c, or a separate PC, tablet, smartphone, or other processing device. As such, FIGURE 2 provides a more functionally-defined, abstract depiction of system 10, where the double-arrows indicate the capabilities of the one or more processors 30 of the controller 22 to communicate, whether over a wired or wireless data connection, with the ventilator and each of the cheek sensors 24a-c. As such, the controller 22 may be located physically on a single device or be distributed across two or more devices. Moreover, the communication between the controller 22 and the cheek sensors 24a-c or ventilator 14 need not be direct. For example, one of the cheek sensors 24a-c may communicate with the controller 22 via the ventilator 14, or even via one of the other cheek sensors 24a-c.

Returning to Fig. 1, in an embodiment, patient interface 12 is a "smart" mask that is equipped with sensors to monitor ventilation therapy, patient status, or other metrics. Such sensors may include microphone 24a and/or PPG sensor 24b located at or near a cheek of patient P when the patient interface 12 is in use. Other sensors may also be incorporated in such a smart mask, including as alternatives to microphone 24a or PPG sensor 24b, such as an accelerometer, flow sensor, gyroscope, temperature sensor, ultrasound transducer, strain sensor, gas sensor, bio-impedance sensor, etc. These sensors can be attached, embedded, or otherwise integrated at various locations in the patient interface, e.g., in the cushion, shell, straps, etc. An advantage of such sensors being integrated into the patient interface is that they offer an opportunity to continuously measure the cheek compliance, resistance, and inertance under different conditions (e.g., patient position, muscle tension).

In an embodiment, microphone 24a is positioned and configured to sense acoustic vibrations from the cheek, or to act as a transducer (both sending and receiving acoustic vibrations), e.g., being placed directly against or very near a portion of the cheek where such vibrations are effectively received. The microphone 24a is particularly configured to acquire an acoustic signal via one or both cheeks while the cheeks are being stimulated by air flowing in and out of the oral cavity during breathing, ventilation, FOT, coughing, gasping for air after an apnea, etc. Based on the frequency spectrum (particularly the harmonics) of the acoustic signal that is acquired by the microphone 24a in this manner, the controller 22 may determine the resonance frequency of the cheeks by performing a Fourier transformation.

Other tools and methods for measuring the resonance frequency may be utilized. For example, the resonance frequency can be determined via pattern recognition in the acoustic signal with a trained algorithm. As another example, if the microphone 24a is configured to act as a transducer, the microphone 24a can apply a frequency sweep to identify the resonance frequency. As another example, an accelerometer or PPG sensor may be used as the cheek sensor 24 to measure cheek vibration instead of microphone 24a. As another example, the signal from a flow sensor coupled with the mechanical ventilator or patient interface may be analyzed, e.g., by spectral analysis or pattern recognition, to isolate the cheek vibration. In any case, a typical resonance frequency of the cheeks is expected to be somewhere in the range 20-40 Hz.

The resonance frequency so determined is a cheek metric based on which the cheek compliance $C_c$ and cheek inertance $I_c$ may be determined. From the resonance frequency f, the product of the cheek compliance $C_c$ and cheek inertance $I_c$ is calculated according to the following equation: $f = 1/(2\pi) * \sqrt{1/C_C I_C}$.

Once the product $C_c I_c$ is calculated, if the cheek compliance $C_c$ is also measured or estimated, the cheek inertance $I_c$ may also be calculated. Alternatively, if the cheek inertance $I_c$ is also measured or estimated, the cheek compliance $C_c$ may be calculated. With both cheek compliance $C_c$ and cheek inertance $I_c$ having been calculated, the cheek impedance $Z_c$ during FOT may be determined according to $Z_c = R_c + i(\omega I_c - E_c/\omega)$, discussed above, where $E_c = 1/C_c$ and $\omega = 2\pi$ (FOT oscillation frequency).

In an embodiment, PPG sensor 24b may be utilized to measure cheek compliance $C_c$ and cheek resistance $R_c$. PPG sensor output is known to be sensitive to skin contact pressure and/or stretch. Normally this is an unwanted effect, however this effect can be utilized to measure cheek deformation. For this purpose, the PPG sensor output needs to be calibrated against cheek inflation volume DV, for example, based on a volunteer study or using a mechanical dummy. The calibration could be determined, for example, by putting on a mask with a PPG sensor positioned against the cheek, and measuring the PPG sensor output while the cheek is being inflated (e.g., with a syringe and with the glottis closed). This gives a calibration curve, volume versus voltage. The measurement could be repeated for different persons and masks. A look-up table or function may then be created where inputs such as PPG output, patient BMI, and/or mask type will output inflation volume DV. Alternatively, a stretchable PPG sensor may be used, where the output depends on strain and, once calibrated, the measured strain relates to cheek expansion DV via basic mechanics or modeling.

The cheek inflation volume DV may then be measured by the PPG sensor 24b under a varying mouth pressure DP during ventilation therapy, providing the cheek compliance, $C_c$ » DV/DP. Here, the pressure at the mouth opening (where air enters the mouth) is the device pressure minus the pressure loss in the tube (flow x tube resistance) assuming zero leak. Typically, when the airflow rate is small and there is no obstruction in the mouth, the difference between the pressure at the mouth opening and the pressure inside the mouth cavity is small. The varying mouth pressure DP therefore can be estimated from the device signals. If there is leak in the system, the DP estimate can be compensated for accordingly using known leak compensation algorithms. The slope of the DP-(DV/dT) curve during therapy then provides the cheek resistance, $R_c$ » DP/(DV/dT), where DV/dT = DQ, the flow in the cheeks.

Other sensors coupled to the cheek, such as a strain sensor, pressure sensor, or compression sensor, are also suitable for similar calibration against cheek inflation volume DV, so that cheek compliance $C_c$ and cheek resistance $R_c$ may be measured.

In an embodiment, cheek metrics can also be determined by fitting system model parameters (e.g., as in Michaelson, 1975 cited above) to the observed sensor data. For example, techniques such as least squares or maximum likelihood may be used, where model inputs may be measured pressure, flow, and cheek movement data (e.g., from sensors in the ventilator 14, patient interface 12, or cheek sensors 24), such that model outputs are cheek compliance $C_c$, resistance $R_c$ and inertance $I_c$. Such a system may be underdetermined, meaning that many solutions exist that can explain a certain pressure-flow relationship, but realistic parameter windows can be defined (e.g., by relying on data such as that in Jaeger, 1982, cited above).

In an embodiment, the cheek sensor 24 signals can be interpreted using a lookup table (LUT). The LUT may be created using a computer simulation of cheek deformation, such as finite element modeling, computational fluid dynamics, or fluid structure interaction. In a Design of Experiment (DOE) approach, the computer model simulates how cheeks with different mechanical properties ($C_c$, $R_c$ and $I_c$) deform and vibrate while being stimulated by air flow. In this application, the LUT inputs may include at least the data from the cheek sensor(s) 24 and the ventilator 14 air flow and pressure sensors. The LUT output may include $C_c$, $R_c$, and/or $I_c$.

In an embodiment, as an alternative to a LUT, a network can be trained with artificial data created by the computer model or with real data from a volunteer study. The trained network inputs may include at least the data from the cheek sensor(s) 24 and the ventilator 14 air flow and pressure sensors. The output may include $C_c$, $R_c$, and/or $I_c$.

In an embodiment, data received from an imaging device 24a cheek sensor is used to determine one or more cheek metrics. Imaging data that is relevant to a patient's cheeks is ubiquitous. For example, pictures from a mobile phone, 3D facial scans, medical imaging, dental x-rays, dedicated image-based patient interface/-mask selector tools, etc., all directly provide information on the geometry of a patient's cheeks. However, to determine cheek compliance $C_c$, resistance $R_c$, and inertance $I_c$, the imaging data may be augmented with a patient-specific biomechanical model. Such a biomechanical model may be utilized to translate one or more cheek geometry parameters determined based on one or more images to a cheek compliance $C_c$ and/or cheek resistance $R_c$.

In an embodiment, a first input of the biomechanical model may be a patient-specific 3D (meshed) geometry of the outer surface of the face, obtained via image processing. The input of the image processing can be a single 2D image, multiple 2D images, or a 3D contour of the face (e.g., an output of a mask selector tool).

A second input of the biomechanical model may be the thickness of the cheek tissue, obtained from literature or measurements. For example, data on the spatial thickness distribution of the cheek tissue, i.e., the muscle and skin composition, is known. *See, e.g.,* Martelly, E., Lee, S., Martinez, K. et al., Development of a Novel Soft Tissue Measurement Device for Individualized Finite Element Modeling in Custom-Fit CPAP Mask Evaluation, Ann Biomed Eng (2024), https://doi.org/10.1007/s10439-024-03581-2. The spatial tissue thickness distribution can be obtained from medical imaging, in which case the thickness distribution is patient specific. *See, e.g.,* Ulusoy, I., Akagunduz, E. and Yirci, M. (2011), Anatomical and dynamic volume spline model applied to facial soft tissue. Comp. Anim. Virtual Worlds, 22: 543-554. Furthermore, techniques used in Forensic Facial Reconstruction can be used to model tissue thickness and muscle thickness from skeletal information, such as dental x-rays.

A third input of the biomechanical model may be the mechanical properties (stiffness, viscosity, mass) of muscles, tendons, and skin - or an equivalent bulk property. Such data can be acquired from literature. *See, e.g.,* Ulusoy (2011), cited above; Petr Kadleĉek and Ladislav Kavan, Building Accurate Physics-based Face Models from Data, Proc. ACM Comput. Graph. Interact. Tech. 2, 2, Article 15 (July 2019).

Based at least on the foregoing inputs, the patient-specific biomechanical model may simulate cheek inflation and deflation while pressurized air is flowing into and out of the oral cavity. The simulation may apply with facial muscles both contracted and relaxed, for example, via the method described by Zhang et al. BioMed Eng OnLine (2016) 15:18. The simulated cheek inflation DV under a slowly varying mouth pressure DP provides the cheek compliance, $C_c$ » DV/DP. The slope of the simulated pressure-flow curve provides the cheek resistance, $R_c$ » P/Q. Inertance can be obtained by simulation of resonating cheeks, per:

$$f = 1/(2\pi) * \sqrt{1/C_C I_C}$$ . The cheek impedance at a certain frequency can also be directly determined by simulating an FOT measurement.

In an embodiment, the biomechanical model is a fluid-structure interaction (FSI) model that simulates the interaction between air flow and cheek deformation, similar to studies that have relied on an FSI model to simulate the deformation of the respiratory tract due to air flow. *See* Shukla, R.K., Srivastav, V.K., Paul, A.R. *et al., Fluid structure interaction studies of human airways, Sādhanā* 45, 229 (2020). In another embodiment, the biomechanical model is a finite element model (FEM) with a time-varying boundary condition (mouth pressure), which simulates the deformation of the cheeks due to varying mouth pressure during inhalation and exhalation. Optionally, the interaction between a patient interface and the cheeks may be incorporated into the biomechanical modeling, particularly is the patient is wearing a full-face mask that may restrict cheek inflation.

In an embodiment, the input for the biomechanical model may be a dynamic image or a series of images of the patient P, such as a video recording (e.g., via a webcam or smartphone "selfie" camera). In addition to tissue thickness estimation that can be obtained from static images, as described above, video provides temporal information. Useful for this technique will be videos of the patient P moving their cheeks, e.g., while speaking, singing, or whistling. Data quality may be enhanced, e.g., by instructing the patient P to record the video while performing a certain procedure like reading a certain text aloud, whistling a certain song, or any other defined behavior that introduces sufficient cheek movement. The combined video images may be analyzed as to particular types or degrees of cheek movement, alone or alongside analysis of the corresponding audio information, to estimate cheek deformation as input to the biomechanical model.

For relatively small (and high frequency) deformation, the foregoing video analysis may benefit from preprocessing such as motion amplification (e.g., as developed by RDI Technologies). These techniques scale up small movements that are only visible in slight intensity changes in pixels to movement over multiple pixels, making it easier to detect and quantify small movements. In any event, once cheek deformation has been estimated based on the video imaging, this information may be input into one or more of the modeling techniques described above for estimating $C_c$, $R_c$, and/or $I_c$.

Optionally, cheek metrics may be determined from the outputs of multiple types of cheek sensors. For example, in addition to using an image capturing cheek sensor (e.g., 24c) to construct a biomechanical model and estimating $C_c$, $R_c$, and/or $I_c$, other cheek sensor data (e.g., via microphone 24a or PPG sensor 24b) may serve as additional inputs for the biomechanical model to adjust the model and resulting estimation. For example, if the patient P changes position overnight or lays on one side so as to compress one cheek, cheek sensors such as a microphone, PPG sensor, or accelerometer may allow these changes to be identified in real time. If one or more sensors are not "free" (e.g., the patient is determined to be lying down, compressing a cheek that is adjacent to the sensor(s)), those sensors may not provide a reliable cheek metric and their measurements could be disabled, disregarded, or weighted appropriately. As another example, if the patient's muscle tension differs from night to night or over longer periods of time that may reflect additional changes such as patient weight loss, again cheek sensors such as a microphone, PPG sensor, or accelerometer can account for such changes in real time. Combining imaging and non-imaging cheek sensors in such manners may be beneficial, for example, where the image data of the patient used for the biomechanical modeling is dated, or is based on fewer or less data-rich images (e.g., a single 2D image vs. a comprehensive 3D scan).

As will be understood, in some instances the data from an imaging cheek sensor (e.g., 24c) and a non-imaging cheek sensor (e.g., 24a, 24b) may be obtained simultaneously, e.g., via a camera being positioned on patient interface 12, on the ventilator 14, or elsewhere in the patient's room or environment, configured to collect one or more images of at least the patient's cheek area(s). In such cases, modification of the biomechanical model may be required, e.g., to address the effect of the patient interface on hindering or otherwise augmenting the cheek movement, and how such effect will influence the imaging data.

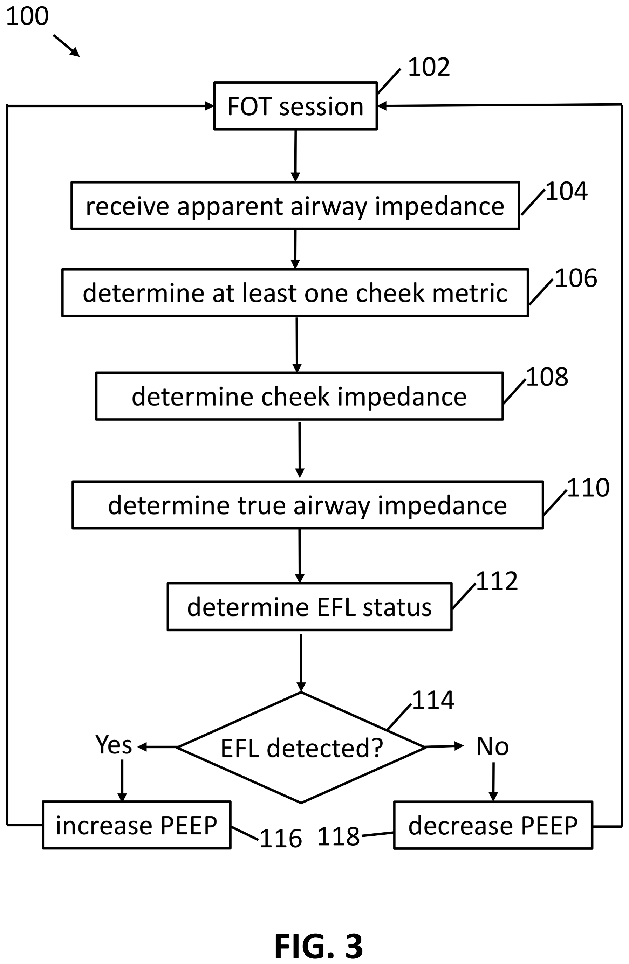

The present invention includes both systems 10 configured to perform the foregoing tasks and methods 100 for putting the invention into practice. FIGURE 3 discloses an example of how the invention may be practiced.

At step 102, an FOT process is performed by the ventilator 14. Based on readings from flow and pressure sensors during the FOT, the ventilator 14 measures the apparent airway impedance. This apparent airway impedance, which actually includes both airway impedance and cheek impedance, is received by the controller 22 at step 104. At step 106, the controller 22 determines at least one cheek metric based on data from at least one of the cheek sensors 22a, 22b, or 22c. At step 108, the controller determines the cheek impedance $Z_c$ based on the one or more cheek metrics. As discussed above, this determination may involve biomechanical modeling based on image information via camera 24a, direct measurements such as cheek pressure or resonance frequency via PPG sensor 24b or microphone 24a, and/or estimations based on direct measurements in combination with a model, lookup table, etc., resulting in outputs of $C_c$, $R_c$, and/or $I_c$ to arrive at the cheek impedance $Z_c$.

With the cheek impedance $Z_c$ known, in step 110 the controller 22 can remove (i.e., subtract) the cheek impedance from the apparent airway impedance to arrive at a true airway impedance. With the true airway impedance known, the controller can determine whether the patient P has EFL at step 112. If EFL is detected (step 114), the controller can output a recommended change to the ventilator settings, such as an increase in PEEP as in step 116. If EFL is not detected, (step 114), the controller can output a recommended change to the ventilator settings, such as a decrease in PEEP as in step 118. Additionally or alternatively, the recommended change to the ventilator settings could be an increase or decrease in respiratory rate at steps 116 or 118, respectively.

For purposes of the present application, outputting a recommended change may include the calculation of a new value of a ventilator setting, the calculation of a degree of change of an existing value, and the storage, transmission/communication, and/or display of the new value or degree of change. For example, a new PEEP setting may be displayed on the ventilator screen as an option for the patient or caregiver to accept, transmitted to a clinician or the patient's electronic medical record for consideration or approval, or communicated to the ventilator as an operational request.

Regardless of whether the outcome 114 regarding EFL status is yes or no, the method 100 may be repeated by initiating another FOT session 102 (ad hoc, at predetermined time intervals, or based on other triggering events such as a detected change in patient status, vital signs, or ventilation sensor outputs) to ensure that the patient's EFL status is known and accurate to optimize the ventilation therapy for the patient on a regular or continuous basis.

As will be appreciated, a wide variety of additions to or modifications of the methods 100 and 200 may be appropriate. For example, the recommended changes to PEEP in steps 116 and 118 may be automatically implemented by the ventilator in a closed-loop fashion. As another example, an alert or other communication about the patient's EFL status or a change in the ventilation parameters on the ventilator 14, may be transmitted to and/or displayed on an appropriate device, such as the ventilator 14, a patient monitor, a smartphone notice, or via an email to the patient's doctor or other message input to a repository for the patient's medical information.

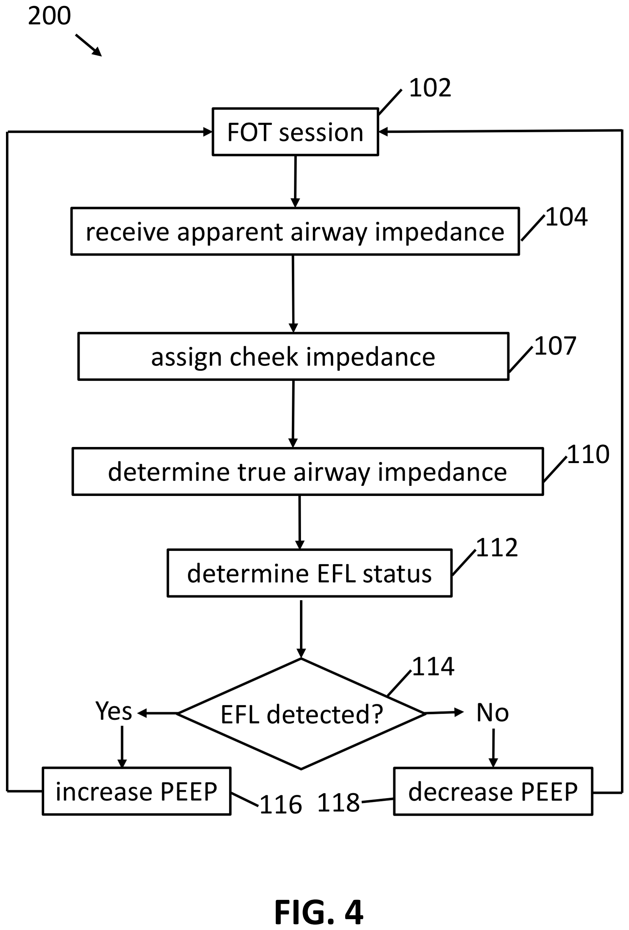

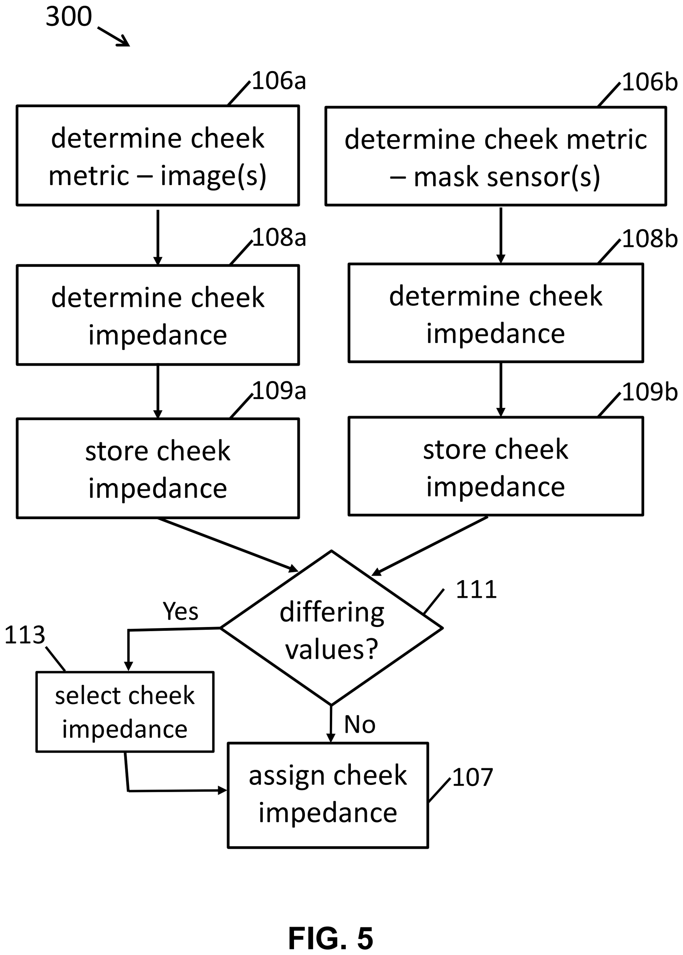

As will be appreciated, the steps of determining at least one cheek metric 106 and determining cheek impedance 108 need not be performed concurrently with the rest of the method 100 if the cheek impedance $Z_c$ is already known from a preexisting measurement. FIGURE 4 shows an exemplary embodiment of a method 200 which is identical to method 100, except that steps 106 and 108 are replaced with the step of assigning cheek impedance 107. This step 107 of assigning a cheek impedance may be performed according to a method 300 depicted in FIGURE 5. At step 106a, image-type cheek metrics are determined by the controller 22. The controller 22 can then determine the cheek impedance $Z_c$ at step 108a and proceed to store the cheek impedance $Z_c$ on a memory at step 109a. Similarly, at step 106b a non-image-based cheek metric (e.g., via sensors 24a, 24b) is determined, from which the cheek impedance $Z_c$ is determined at step 108b and then stored at step 109b.

The method 300 includes a decision step 111 for determining how to handle the existence of multiple stored cheek impedance $Z_c$ values. For example, if there is only one cheek impedance $Z_c$ value stored (via step 109a or 109b), there would be no inconsistent or differing values per decision step 111, and the single stored cheek impedance value will be assigned in step 107. This situation may arise, for example, if the only cheek impedance measurement for the patient P comes via an image-based cheek metric (step 106a) that was collected when the patient P was fitted for a patient interface 12 using a mask selector tool (and such a tool's collection of 2D and/or 3D information about the patient's face).

If, subsequently, while the patient P is using the patient interface 12, non-image-based cheek metrics are determined (step 106b) that result in a second cheek impedance value being stored (109b), the controller 22 may determine at step 111 whether the first and second cheek impedance values are the same. If so, the second value (which equals the first value) will be assigned at step 107. If, however, the second value differs from the first value, the controller may select at step 113 which cheek impedance value should be used. This process may be used to choose between multiple stored cheek impedances 109a or 109b of the same type-e.g., choosing which of two microphone-based cheek impedance values should be assigned, or whether an ultrasound-based cheek impedance value should be chosen over a value determined from a single "selfie" photo.

As will be appreciated, this selecting step 113 may be based on one or multiple factors including, e.g., a determination that the second value: diverges from the first value by a predetermined amount or percentage (e.g., 1%, 5%, or 10%), has deviated from the first value and remained stable over a predetermined period of time, is more recent than the first value, is based on a more reliable cheek metric, is based on a more reliable cheek impedance model, etc. The selecting step 113 need not be an either/or decision between only two values, but may be a blended value of two or more values based on relative weights of various foregoing factors, a sliding-window average, a least-square fit of various data points, etc. The selected cheek impedance value per step 113 may also be stored in the memory for comparison to future cheek impedance values (stored per 109a, 109b) to perform steps 111 and 113 again and update the selected cheek impedance value. In any case, once the cheek impedance $Z_c$ has been selected, it may be assigned per step 107 and relied on within process 200 in Fig. 4. to determine true airway impedance etc. The method 300 may also be repeated (ad hoc, at predetermined time intervals, or based on other triggering events such as new cheek impedance values being stored) so that the cheek impedance $Z_c$ remains current and accurate as time elapses and/or new information becomes available.

As will be appreciated, the performance of any steps in methods 100, 200, or 300, may be triggered to be performed automatically based on the initiation or completion of another step. For example, whenever an FOT session is initiated or completed 102, step 106 may be triggered to determine at least one cheek metric 106 (which may in turn trigger step 108 to determine cheek impedance), or step 107 may be triggered to assign cheek impedance 107. As another example, if a new image-based cheek metric is determined 106a or stored 109a, a mask sensor-based cheek metric may be triggered to be determined 106b (or vice versa), and then one or more of cheek impedance determinations 108a-b, storage 109a-b, comparison 111, selection 113, and assignment 107 may be triggered to follow automatically thereafter.

As will be appreciated, steps of methods 100, 200, or 300 may also automatically trigger other actions outside the specific steps shown in Figs. 3-5, and actions outside the steps shown in Figs. 3-5 may automatically trigger steps within the methods 100, 200, of 300. For example, to conserve power for cheek sensors 24, controller 22 may be configured to change the operational status of one or more components of the cheek sensors 24 (e.g., on/off, high-power/low-power) based on the FOT session 102 beginning or ending, so that the sensors 24 are only powered as necessary to provide data to the controller 22 to allow the controller 22 to determine the associated cheek metric(s). As another example, an image-based cheek metric may be automatically determined 106a upon detection of a new medical image in a patient's electronic medical records.

As will be appreciated, although the foregoing examples have involved systems and methods relying on obtaining airway impedance via an FOT session 102, the systems and methods described herein for determining cheek impedance are suitable for use in other impedance estimation process or procedure, i.e., in any context where it is desirable to have accurate measurements and/or estimates of cheek impedance for its own sake, or as part of an effort to determine true airway impedance by removing the effect of cheek impedance from an apparent airway impedance measurement or estimation.

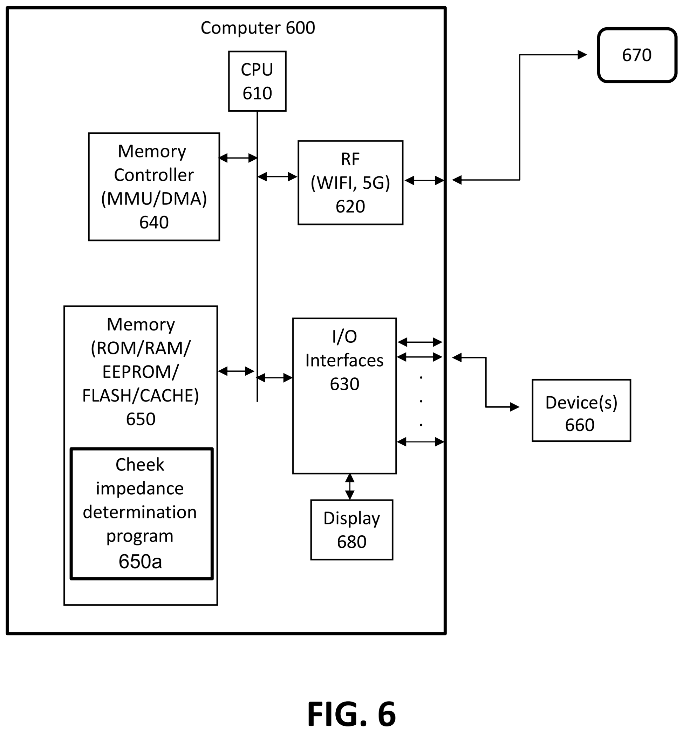

Referring now to FIGURE 6, as discussed more briefly above, it will be readily understood that certain embodiments can be implemented using any of a wide variety of devices or combinations of devices and components. In FIG. 6 an example of a computer 600 and its components are illustrated, which may be used in a device for implementing the functions or acts described herein, e.g., forming and training a biomechanical model, processing sensor input, employing a lookup table, applying ventilator setting adjustments, etc. Also, circuitry other than that illustrated in FIG. 6 may be utilized in one or more embodiments. The example of FIG. 6 includes certain functional blocks, as illustrated, which may be integrated onto a single semiconductor chip to meet specific application requirements.

One or more processing units are provided, which may include a central processing unit (CPU) 610, one or more graphics processing units (GPUs), and/or micro-processing units (MPUs), which include an arithmetic logic unit (ALU) that performs arithmetic and logic operations, instruction decoder that decodes instructions and provides information to a timing and control unit, as well as registers for temporary data storage. CPU 610 may comprise a single integrated circuit comprising several units, the design and arrangement of which vary according to the architecture chosen.

Computer 600 also includes a memory controller 640, e.g., comprising a direct memory access (DMA) controller to transfer data between memory 650 and hardware peripherals. Memory controller 640 includes a memory management unit (MMU) that functions to handle cache control, memory protection, and virtual memory. Computer 400 may include controllers for communication using various communication protocols (e.g., I$^2$C, USB, etc.).

Memory 650 may include a variety of memory types, volatile and nonvolatile, e.g., read only memory (ROM), random access memory (RAM), electrically erasable programmable read only memory (EEPROM), Flash memory, and cache memory. Memory 650 may include embedded programs, code, and downloaded software, e.g., cheek impedance determination pgoram 650a that provides coded methods such as illustrated and described in connection with Figs. 3, 4, and 5 (or parts thereof). Memory 650 may also include an operating system, application programs, other program modules, code, and program data, which may be downloaded, updated, or modified via remote devices. Memory 650 may also store the cheek impedance values described above with reference to Fig. 5.

A system bus permits communication between various components of the computer 600. I/O interfaces 630 and radio frequency (RF) devices 620, e.g., WIFI and telecommunication radios, may be included to permit computer 600 to send data to and receive data from remote devices using wireless mechanisms, noting that data exchange interfaces for wired data exchange may be utilized. Computer 600 may operate in a networked or distributed environment using logical connections to one or more other remote computers or devices 670, such as a ventilator. The logical connections may include a network, such local area network (LAN) or a wide area network (WAN) but may also include other networks/-buses. For example, computer 600 may communicate data with and between device(s) 660, for example mechanical ventilators 14, cheek sensors 24, patient monitors, user device(s) such as PCs, tablets, and smartphones, database(s) storing electronic medical records, servers storing biomechanical models that respond to application programming interface (API) calls, etc.

Computer 600 may therefore execute program instructions or code configured to obtain, store, and analyze patient medical data and perform other functionality of the embodiments, such as described in connection with Figs. 3, 4, and 5. A user can interface with (for example, enter commands and information) the computer 600 through input devices, which may be connected to I/O interfaces 630. A display 680 or other type of output device may be connected to or integrated with the computer 600, for example via an interface selected from I/O interfaces 630.

It should be noted that the various functions described herein may be implemented using instructions or code stored on a memory, e.g., memory 650, that are transmitted to and executed by a processor, e.g., CPU 610. Computer 600 includes one or more storage devices that persistently store programs and other data. A storage device, as used herein, is a non-transitory computer readable storage medium. Some examples of a non-transitory storage device or computer readable storage medium include, but are not limited to, storage integral to computer 600, such as memory 650, a hard disk or a solid-state drive, and removable storage, such as an optical disc or a memory stick.

Program code stored in a memory or storage device may be transmitted using any appropriate transmission medium, including but not limited to wireless, wireline, optical fiber cable, RF, or any suitable combination of the foregoing.

Program code for carrying out operations according to various embodiments may be written in any combination of one or more programming languages. The program code may execute entirely on a single device, partly on a single device, as a stand-alone software package, partly on single device and partly on another device, or entirely on the other device. In an embodiment, program code may be stored in a nontransitory medium and executed by a processor to implement functions or acts specified herein. In some cases, the devices referenced herein may be connected through any type of connection or network, including a local area network (LAN) or a wide area network (WAN), or the connection may be made through other devices (for example, through the Internet using an Internet Service Provider), through wireless connections or through a hard wire connection, such as over a USB connection.

An embodiment may be implemented in a variety of devices, including ventilators, patient monitors, "smart" patient interfaces, PCs, or other user devices such as a smartphone or tablet running a mobile application. In one embodiment, referring to Fig. 3, the steps of obtaining at least one cheek metric and determining a cheek impedance may be performed locally on medical device processor or on a user device processor, such as a PC or smartphone. In such an embodiment, a program or model may be downloaded and run locally on the medical device or user device. For example, a cheek impedance determination program 650a as described herein may include a model and one or more application programming interfaces (APIs) to call an appropriate subsystem of the device. In some embodiments, the model may be selected (e.g., downloaded, activated for running, etc.) based on identified characteristics or contextual data, for example by a user or as part of an application routine, based on data availability, etc. For less processor-intensive applications, such as those relying only on cheek sensors within a smart mask (e.g., 24a, 24b), the program 650a may run frequently or continuously on the medical devices or user devices. In more processing-intensive embodiments, such as biomechanical modeling for image-based cheek metrics, the model may run on a dedicated server or in the cloud to conserve computing resources locally.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

The invention has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A system for determining airway impedance of a patient undergoing mechanical ventilation, comprising:
   a controller including one or more electronic processors configured to:

   receive an apparent airway impedance of a patient measured during an impedance estimation procedure by a mechanical ventilator;
   determine at least one cheek metric based on data received from at least one cheek sensor associated with the patient;
   determine a cheek impedance based on the at least one cheek metric; and
   determine a true airway impedance based on the apparent airway impedance and the cheek impedance.

2. The system of claim 1, wherein the at least one cheek sensor comprises a microphone.

3. The system of claim 2, wherein the at least one cheek metric comprises a resonance frequency.

4. The system of claim 1, wherein the at least one cheek sensor is a PPG sensor.

5. The system of claim 4, wherein the at least one cheek metric comprises a change in volume.

6. The system of claim 1, wherein the at least one cheek sensor comprises an imaging device and the at least one cheek metric comprises at least one cheek geometry parameter.

7. The system of claim 6, wherein the controller is configured to determine cheek impedance based on a biomechanical model, wherein the biomechanical model is based on the at least one cheek geometry parameter.

8. The system of claim 1, wherein the controller is further configured to recommend a change to at least one ventilation parameter based on the true airway impedance.

9. The system of claim 8, wherein the change to at least one ventilation parameter is an increase or decrease in positive end expiratory pressure (PEEP).

10. A method for determining airway impedance of a patient undergoing mechanical ventilation, by a controller including one or more processors, comprising the steps of:

  receiving an apparent airway impedance of a patient measured while the patient is undergoing an impedance estimation procedure by a mechanical ventilator;
  receiving data from at least one cheek sensor associated with the patient;
  determining at least one cheek metric based on the data of at least one cheek sensor;
  determining a cheek impedance based on the at least one cheek metric; and
  determining a true airway impedance based on the apparent airway impedance and the cheek impedance.

11. The method of claim 10, further comprising the step of determining an expiratory flow limitation (EFL) status based on the true airway impedance.

12. The method of claim 11, further comprising the step of outputting a recommended change in positive end expiratory pressure (PEEP) level based on the EFL status.

13. The method of claim 10, wherein the at least one cheek sensor comprises a microphone, and wherein the cheek metric comprises a resonance frequency.

14. The method of claim 10, wherein the at least one cheek sensor comprises a PPG sensor, and wherein the cheek metric comprises a change in volume.

15. The method of claim 10, wherein the at least one cheek sensor comprises an imaging device, the at least one cheek metric comprises at least one cheek geometry parameter, and the cheek impedance is determined based on a biomechanical model, wherein the biomechanical model is based on the at least one cheek geometry parameter.

**FIG. 1**

10

Ventilator 14

Controller 22

Processor(s)

| 30 | 30 | 30 |

Cheek Sensor 24a

Cheek Sensor 24b

Cheek Sensor 24c

**FIG. 2**

100

102

FOT session

receive apparent airway impedance — 104

determine at least one cheek metric — 106

determine cheek impedance — 108

determine true airway impedance — 110

determine EFL status — 112

114

Yes ← EFL detected? → No

increase PEEP — 116   118 — decrease PEEP

**FIG. 3**

200

102 FOT session

104 receive apparent airway impedance

107 assign cheek impedance

110 determine true airway impedance

112 determine EFL status

114 EFL detected?

Yes → 116 increase PEEP

No → 118 decrease PEEP

**FIG. 4**

300

106a

determine cheek
metric – image(s)

106b

determine cheek metric
– mask sensor(s)

108a

determine cheek
impedance

108b

determine cheek
impedance

109a

store cheek
impedance

109b

store cheek
impedance

111

differing
values?

Yes

113

select cheek
impedance

No

assign cheek
impedance

107

**FIG. 5**

## Computer 600

CPU
610

670

Memory
Controller
(MMU/DMA)
640

RF
(WIFI, 5G)
620

Memory
(ROM/RAM/
EEPROM/
FLASH/CACHE)
650

I/O
Interfaces
630

Device(s)
660

Cheek
impedance
determination
program
650a

Display
680

# FIG. 6

## EUROPEAN SEARCH REPORT

**Application Number**

EP 25 21 4778

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2010/147305 A1 (DELLACA RAFFAELE [IT] ET AL) 17 June 2010 (2010-06-17) * paragraph [0001] - paragraph [0010] * * paragraph [0017] - paragraph [0072] * * figures 1-4b * | 1-15 | INV. A61M16/00 G16H20/00 A61B5/08 ADD. A61M16/06 |
| A,D | MICHAELSON E D ET AL: "Pulmonary mechanics by spectral analysis of forced random noise.", JOURNAL OF CLINICAL INVESTIGATION, vol. 56, no. 5, 1 November 1975 (1975-11-01), pages 1210-1230, XP093376844, ISSN: 0021-9738, DOI: 10.1172/jci108198 * abstract * * sections "Introduction", "Methods", "Results", "Discussion" and "Appendix", in particular Fig. 1 and 15 * | 1-15 | |
| A | CN 117 731 269 A (MEHOW INNOVATIVE LTD) 22 March 2024 (2024-03-22) * paragraph [0001] - paragraph [0041] * * paragraph [0056] - paragraph [0145] * * figures 1-11 * | 1-15 | TECHNICAL FIELDS SEARCHED (IPC) A61M A61B G16H |

The present search report has been drawn up for all claims

| Place of search | Date of completion of the search | Examiner |
|---|---|---|
| Munich | 13 March 2026 | Aguado, Miguel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 4778

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2026

| Patent document cited in search report | Publication date | Patent family member(s) | Publication date |
|---|---|---|---|
| US 2010147305 A1 | 17-06-2010 | NONE | |
| CN 117731269 A | 22-03-2024 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JUNHASAVASDIKUL, D et al.** Expiratory Flow Limitation During Mechanical Ventilation. *CHEST 2018*, 2018, vol. 154 (4), 948-962
- **JUNHASAVASDIKUL, D et al.** Expiratory flow limitation during mechanical ventilation: real-time detection and physiological subtypes. *Crit Care*, 2024, vol. 28, 171
- **DELLACA R.L et al.** Detection of expiratory flow limitation in COPD using the forced oscillation technique. *Eur. Respir J 2004*, 2004, vol. 23, 232-240
- **MICHAELSON, E.D. et al.** Pulmonary Mechanics by Spectral Analysis of Forced Random Noise. *The Journal of Clinical Investigation Volume*, November 1975, vol. 56, 1210-1230
- **JAEGER, M.J.** Effect Of The Cheeks And The Compliance Of Alveolar Gas On The Measurement Of Respiratory Variables. *Respiration Physiology*, 1982, vol. 47, 325-340
- **CLEMENTS, J.A et al.** Estimation Of Pulmonary Resistance By Repetitive Interruption Of Airflow. *J Clin Invest.*, July 1959, vol. 38 (7), 1262-70
- **MARTELLY, E ; LEE, S. ; MARTINEZ, K et al.** Development of a Novel Soft Tissue Measurement Device for Individualized Finite Element Modeling in Custom-Fit CPAP Mask Evaluation. *Ann Biomed Eng*, 2024, https://doi.org/10.1007/s10439-024-03581-2
- **ULUSOY, I ; AKAGUNDUZ, E. ; YIRCI, M**. Anatomical and dynamic volume spline model applied to facial soft tissue.. *Comp. Anim. Virtual Worlds*, 2011, vol. 22, 543-554
- **LADISLAV KAVAN**. Building Accurate Physics-based Face Models from Data. *Proc. ACM Comput. Graph. Interact. Tech*, July 2019, vol. 2, 2
- **ZHANG et al.** *BioMed Eng OnLine*, 2016, vol. 15, 18